Europäisches Patentamt

European Patent Office

Office européen des brevets

(19)

(11) Publication number: **0 218 892 B1**

# EUROPEAN PATENT SPECIFICATION

(45) Date of publication of patent specification: **22.05.91**

(51) Int. Cl.⁵: **A61L 9/12, A61L 9/04**

(21) Application number: **86112248.9**

(22) Date of filing: **04.09.86**

(54) Device for dispensing volatile substances.

(30) Priority: **15.10.85 US 787069**

(43) Date of publication of application:
**22.04.87 Bulletin 87/17**

(45) Publication of the grant of the patent:
**22.05.91 Bulletin 91/21**

(84) Designated Contracting States:
**CH DE FR GB IT LI NL**

(56) References cited:
**EP-A- 0 019 010**
**US-A- 4 413 779**

(73) Proprietor: **Union Camp Corporation**
**1600 Valley Road**
**Wayne New Jersey 07470 - 2066(US)**

(72) Inventor: **Locko, George A.**
**41 Wolfpack Road**
**Trenton New Jersey 08619(US)**

(74) Representative: **Finck, Dieter et al**
**Patentanwälte v. Füner, Ebbinghaus, Finck**
**Mariahilfplatz 2 & 3**
**W-8000 München 90(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a device for dispensing volatile substances.

The device is used for the slow release of a volatile substance such as a fragrance, insect repellant, deodorant, medicament and the like.

Articles of many types for the controlled release of volatile substances to the environment are well known in the art. However, all of the articles which have heretofore been disclosed in the art suffer from various disadvantages, especially with respect to performance. Ideally, an article for dispensing a fragrance or other volatile substance should dispense the volatile substance at an essentially constant rate over an extended period of time to provide an effective level of the volatile substance in the environment, up to the point at which the fragrance contained within the article is depleted.

Gel-type deodorizer dispensers in which a fragrance is dispersed in a water-based gel are generally useful in that they provide acceptable levels of fragrance to the environment. However, the gel-type systems suffer from the disadvantage that the lifetimes for fragrance release are too short, typically on the order of one or two weeks due to the rapid evaporation of water and fragrance from the gel formulations. An additional disadvantage of the gel-type systems is that only certain fragrance compositions which are compatible with the water-based gels, can be used in the gel formulations.

Articles comprised of fragrances dispersed in various plastics such as polyamides, ethylene-vinyl acetate copolymer, cross-linked methacrylate derivatives, and the like have been described. Although these articles have seen use, the plastic articles suffer from the disadvantage that the levels of fragrance output are frequently too low to be effective and these articles provide non-linear release of fragrance, that is, the level of the fragrance output is typically high during the initial period of use, but then drops within a short period of time to an almost imperceptible level. An additional disadvantage of the plastic articles is that many fragrance substances are incompatible with the plastics. This frequently results in sweating of the plastic, a situation where beads of fragrance material appear on the surface of the plastic. A further disadvantage of the articles in which the fragrances are dispersed in the plastics is that the liquid fragrances must either be admixed with the plastic by melt blending at high temperatures or mixed into the monomer prior to carrying out the polimerization reaction. In both instances, substantial degradation of fragrances occur. Examples of plastic articles of the aforementioned types are described in U.S.-A- 4,095,031; 4,411,855; 3,926,655; 4,184,009 and CA-A- 1,099,429.

JP-A- 82-40,558 describes a fragrant, rubber-like molding material, formed by dispersing a fragrance in a silicone rubber and then carrying out a cross-linking reaction with an organometal salt. The articles suffer from the disadvantage that severe sweating of the silicone rubber occurs at even moderate loadings of volatile substances due to incompatibility of the silicone polymer and the volatile substances.

Wick-type deodorizers have also been described in the prior art. Although these reservoir-type systems are effective under limited conditions, the dispensers are objectionable in appearance. The wicks are sometime prone to clogging and require special deodorants which are suitable for water-based formulations.

The U.S.-A- 4,161,283 describes a fragrance-dispensing pouch which is comprised of a fragrance-containing reservoir defined by an outer wall made of a fragrance-permeable film, which is heat sealed to an impermeable inner wall by an adhesive substance. This device suffers from the disadvantage that it is not self-supporting and therefore cannot be conveniently located where desired. It is also difficult to manufacture and an article of this type cannot be made into a variety of desirable shapes.

In view of the foregoing, the object of this invention is to provide a self-supporting device for dispensing or releasing volatile substances from within the device to the surrounding environment at a relatively constant rate and at an effective level until the point in time at which the volatile substance contained within the device has been essentially completely released.

According to the invention, this object is obtained with a device for dispensing volatile substances which comprises an open-ended hollow body of a material which is substantially impermeable to said volatile substance; a liquid volatile substance contained in the hollow of said body; a silicone rubber closure mounted in the open end of the hollow body and in contact with the liquid volatile substance, said closure being permeable to said substance and a base attached to the open end of the hollow body, said base including means to allow circulation of air below the silicone rubber closure.

Preferably the silicone rubber closure has a thickness of from 2 to 15 mm. The volatile substance-dispensing device according to the invention can be made in a variety of shapes, can release almost all of the volatile substance, results in a cost-effective use of the volatile substance, and can be used with almost any fragrance formulation or other volatile substance, including expensive perfumes which can be used without any alteration of composition or aroma character of the volatile substance, since the volatile substance is added to the reservoir after the hollow body has been made. Further the reservoir can be refilled with either the same or another volatile substance after the original volatile substance has been depleted. This is attractive to the typical consumer due to its versatility and a perceived cost savings. It is attractive to the supplier of the volatile substance, for example, a fragrance house, because it maximizes resale of their product to the original consumer.

For a better understanding of the invention as well as other objects and further features thereof, reference is made to the following detailed description to be read in conjunction with the accompanying drawings, wherein:

Fig. 1    is a perspective view of the device and

Fig. 2    is the axial-sectional view 2-2 of Fig. 1.

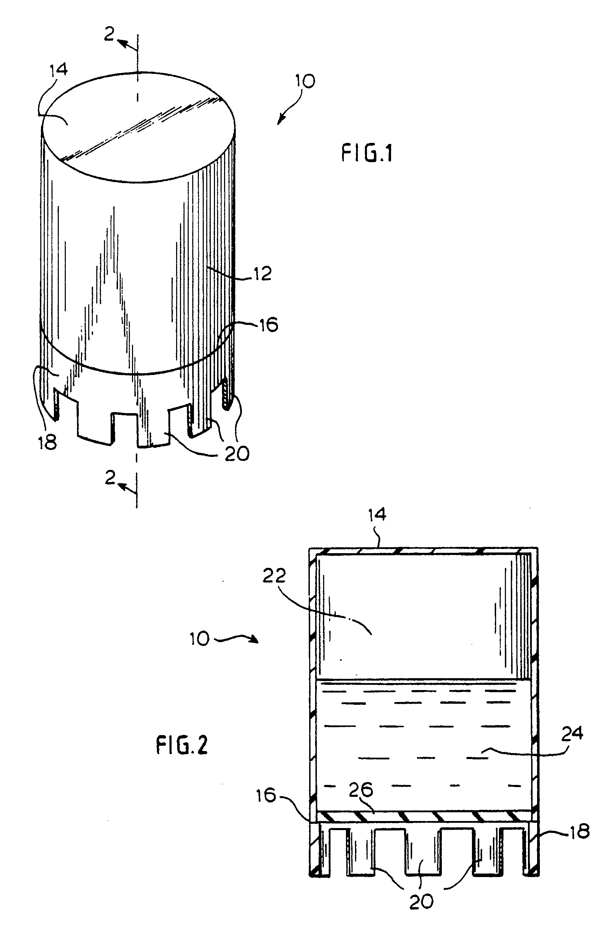

The device 10 as shown in Fig. 1 comprises a cylindrical body 12 which may be fabricated from any conventional material such as metal, glass, synthetic polymeric resin and like materials, which are impermeable to the volatile substance to be contained and dispensed. The body 12 has a closed end 14 and an open end 16 resting on a base cap 18 which is supported by legs 20 about the lower periphery of the base cap 18.

As shown in Fig. 2, the device 10 includes a chamber 22 defined by the walls of the body 12 and the ends 14, 16. The chamber 22 is filled or partially filled with a liquid volatile substance 24. A silicone rubber disc 26 is affixed to the open end 16 of the hollow body 12 and held tightly in place by the cap 18 which serves as a base for the device 10 and is also designed to allow circulation of air below the silicone rubber disc 26, between legs 20.

The hollow body 12 is preferably constructed of a glass, plastic or metal material and is substantially non-permeable to the liquid volatile substance.

Typically, the hollow body 12 of the device 10 is designed to contain between about 1 to 50 g of a liquid volatile substance, preferably between about 3 to 25 g. Representative of fragrances and other volatile substances which may be held in the device 10 are liquid perfumes, pesticides, odorants, insecticides, medicaments, fragrances, deodorants, insect repellants and the like.

The silicone rubber disc 26 is permeable to the contained volatile substance 24 and permits a slow, controlled release of substance 24 to the surface of the rubber disc 26 where the substance 24 volatilizes and is carried away by air circulating between the legs 20.

Unexpectedly, it has been found that the silicone rubber disc or slab 26 thickness is not a limiting variable, in that silicone rubber slabs having very thick walls, as much as 5 mm to 15 mm, will give high rates of output of volatile substances which can permeate silicone rubber plugs. The permeation is constant and at a uniform rate over a relatively long period of time.

Silicone rubber discs or slabs 26 thin as 2 to 3 mm, may also be used in the devices 10 of the invention. However, as slab thickness is reduced, the silicone rubber slab may deform due to plasticization or other effects by the liquid volatile substance and due to the weight of the liquid volatile substance 25 above it. Therefore, a disc or slab thickness of 5 mm to 15 mm is preferred.

The use of silicone rubbers for disc 26 in the invention is particularly important, because the gas permeability of the silicone rubbers allows rapid release of the volatile substances from within the hollow body. In accordance with this invention, a silicone rubber is herein defined generally as a cross-linked silicone elastomer of the type vulcanized at room temperature (RTV) or at elevated temperature (HTV). Dimethyl siloxanediol with silicone resin or alkyl silicate as cross-linking agents are typically used. Typically, fillers such as silica, calcium carbonate, titanium oxide, and the like are normally added to the polymer formulation, usually by the manufacturer of the silicone polymer, as the filler materials provide rigidity.

One example of a silicone rubber which is advantageously used in the invention is an RTV silicone rubber. This material can be cross-linked at room temperature with an RTV catalyst. When allowed to cure in a suitable mold, this material will give a disc or slab 26 which can then be affixed to a hollow body 12, loaded with a liquid volatile and used as described herein.

Thus, according to the invention to the open end of the hollow body 12 a disc 26 made of silicone rubber is affixed by compression.

The device comprises the reservoir for containing the volatile substance, the silicone rubber disc, and a screw cap which functions to tightly hold the silicone rubber disc in place and also functions as a base through which air is allowed to circulate by virtue of the base design.

## Claims

1.  A device for dispensing volatile substances which comprises;

    A. an open-ended hollow body (12) of a material which is substantially impermeable to said volatile substance;

    B. a liquid volatile substance (24) contained in the hollow of said body (12);

C. a silicone rubber closure (26) mounted in the open end (16) of the hollow body (12) and in contact with the liquid volatile substance (24), said closure (26) being permeable to said volatile substance (24) and

D. a base (18) attached to the open end (16) of the hollow body (12), said base (18) including means (20) to allow circulation of air below the silicone rubber closure (26).

2. The device according to claim 1 wherein the silicone rubber closure (26) has a thickness of from 2 to 15 mm.


**Revendications**

1. Dispositif pour libérer des substances volatiles, qui comprend :

A. un corps creux (12) à extrémité ouverte, en une matière qui est essentiellement imperméable à la substance volatile,

B. une substance volatile liquide (24) contenue dans la cavité de ce corps (12),

C. un élément d'obturation (26), en caoutchouc de silicone, monté dans l'extrémité ouverte (16) du corps creux (12) et en contact avec la substance volatile liquide (24), cet élément d'obturation (26) étant perméable à cette substance volatile (24), et

D. un élément de base (18) fixé à l'extrémité ouverte (16) du corps creux (12), cet élément de base (18) comportant des moyens (20) permettant une circulation de l'air au-dessous de l'élément d'obturation (26) en caoutchouc de silicone.

2. Dispositif suivant la revendication 1, dans lequel l'élément de fermeture (26) en caoutchouc de silicone a une épaisseur de 2 à 15 mm.


**Ansprüche**

1. Vorrichtung zum Abgeben flüchtiger Substanzen, welche aufweist:

A. einen offenendigen hohlen Körper (12) aus einem Material, welches für die flüchtige Substanz im wesentlichen undurchlässig ist;

B. eine flüssige flüchtige Substanz (24), die in dem Hohlraum des Körpers (12) enthalten ist;

C. einen Silikon-Kautschuk-Verschluß (26), der in dem offenen Ende (16) des hohlen Körpers (12) angeordnet ist und in Kontakt mit der flüssigen flüchtigen Substanz (24) steht, wobei der Verschluß (26) für die flüchtige Substanz (24) durchlässig ist und

D. eine Basis (18), die an dem offenen Ende des hohlen Körpers (12) befestigt ist, wobei die Basis (18) Einrichtungen (20) aufweist, welche eine Zirkulation der Luft unter dem Silikon-Kautschuk-Verschluß (26) erlauben.

2. Vorrichtung nach Anspruch 1, bei welcher der Silikon-Kautschuk-Verschluß (26) eine Dicke von 2 bis 15 mm hat.

FIG.1

FIG.2